# EUROPÄISCHE PATENTANMELDUNG

(11) **EP 2 514 906 A2**
(43) Veröffentlichungstag der Anmeldung: **24.10.2012**
(21) Anmeldenummer: 12450005.9
(22) Anmeldetag: 26.01.2012
(51) Int. Cl.: E06B 3/673

(54) **Verfahren zum Zusammenbau von Isolierglas-Rohlingen**

(30) Priorität: 18.04.2011 AT 5432011
(71) Anmelder: Inova Lisec Technologiezentrum GmbH, 3353 Seitenstetten (AT)
(72) Erfinder: Mader, Leopold, 3364 Neuhofen/Ybbs (AT)
(74) Vertreter: Beer, Manfred

(57) **Zusammenfassung**

Um beim Zusammenbauen von Isolierglasrohlingen (6), bei welchen der Abstandhalter (3) beidseitig mit einem Strang (4) aus einem wärmeaktivierbaren Kleber belegt ist, auszuführen, wird der Kleber dadurch auf seine Aktivierungstemperatur erwärmt, dass der Abstandhalter (3) durch Anlegen von Spannung nach Art einer elektrischen Widerstandsheizung erwärmt wird.

## Beschreibung

Die Erfindung betrifft ein Verfahren zum Zusammenbauen von Isolierglas-Rohlingen, bei dem die Glastafeln mit dem Abstandhalter durch einen wärmeaktivierbaren Kleber, insbesondere einen Hot-Melt-Kleber, verbunden sind.

Bislang ist ein Zusammenbau von Isolierglas-Rohlingen der oben genannten Gattung so vorgegangen worden, dass das Paket aus zwei oder drei Scheiben mit dazwischen-gefügtem Abstandhalter bzw. Abstandhaltern in eine Heizpresse eingebracht wurde und nach dem Erhitzen der Glastafeln und des Klebers auf seine Aktivierungstemperatur zusammengepresst wurde, um die Glastafeln in Anlage an den erwärmten Kleber zu bringen.

Diese Arbeitsweise hat den Nachteil, dass im Innenraum des Isolierglases beim Abkühlen Unterdruck entsteht, was ein konkaves Wölben der Glastafeln zur Folge hat.

Ein weiterer Nachteil besteht darin, dass bei dieser Arbeitsweise ein Füllen des Isolierglases mit einem von Luft verschiedenen Gas nicht möglich ist.

Der Erfindung liegt die Aufgabe zu Grunde, ein Verfahren der eingangs genannten Gattung zur Verfügung zu stellen, mit dem Isolierglas der eingangs genannten Gattung problemlos hergestellt werden kann.

Gelöst wird diese Aufgabe erfindungsgemäß mit einem Verfahren, welches die Merkmale von Anspruch 1 aufweist.

Bevorzugte und vorteilhafte Ausgestaltung der Erfindung sind Gegenstand der Unteransprüche.

Da beim erfindungsgemäßen Verfahren nicht das gesamte Scheibenpaket erwärmt wird, indem es in eine erwärmte Presse eingebracht wird, sondern nur der Abstandhalter, treten die oben genannten Probleme bekannter Arbeitstechniken nicht auf.

Weitere Einzelheiten und Merkmale des erfindungsgemäßen Verfahrens ergeben sich aus der nachstehenden Beschreibung an Hand der schematischen Zeichnungen. Es zeigt:

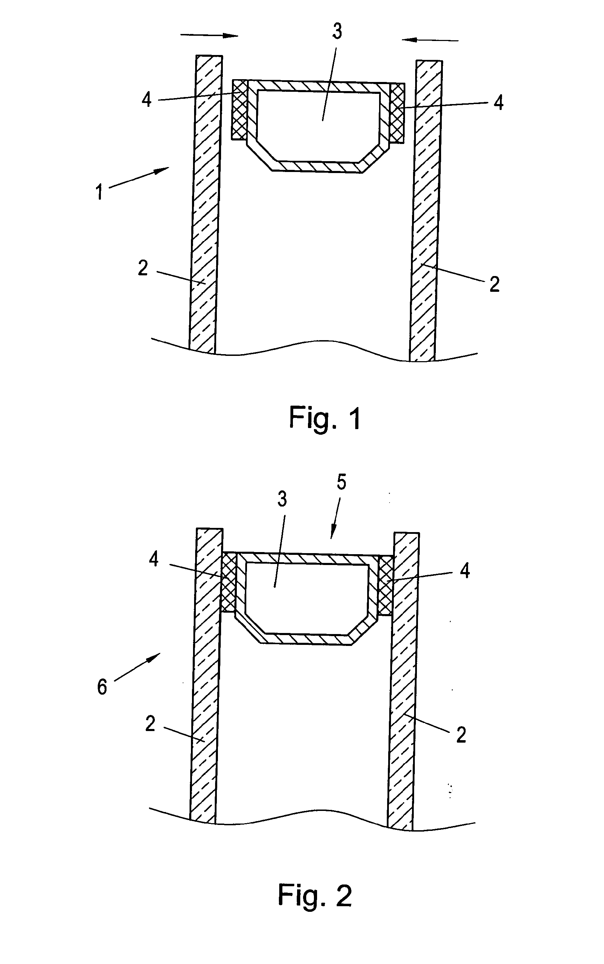

- Fig. 1: ein Paket aus Glasscheiben und Abstandhalter vor dem Zusammenbau eines Isolierglasrohlings und
- Fig. 2: einen Isolierglasrohling nach dem Zusammenbau nach dem erfindungsgemäßen Verfahren,
- Fig. 3: eine erste Variante, wie an einen Abstandhalter Spannung angelegt werden kann und
- Fig. 4: eine zweite Variante, wie an einen Abstandhalter Spannung angelegt werden kann.

Ein Paket 1 für das Herstellen eines Isolierglasrohlings besteht aus zwei Glasscheiben 2 (Glastafeln) und einem dazwischengefügten Abstandhalter 3. Beide Seitenflächen der Abstandhalters 3, die den Glasscheiben 2 zugekehrt sind, sind mit einer Schicht 4 (Strang) aus durch Wärme aktivierbaren Kleber belegt, der z.B. ein Hot-Melt-Kleber ist. Dieses Paket wird in eine Presse für den Zusammenbau von Isolierglasrohlingen 1 eingebracht. Diese Presse kann gleichzeitig eine Presse sein, in welcher der Isolierglasrohling mit einem von Luft verschiedenen Gas (z.B. ein Edelgas) gefüllt wird.

Um den Abstandhalter 3 (aus elektrischen Strom leitenden Metall oder wenigstens teilweise aus Strom leitenden Metall)zu erwärmen und damit auch die auf ihm aufgetragenen Kleberstränge 4, um diese durch Wärme zu aktivieren, wird dieser an eine Stromquelle angeschlossen, so dass dieser durch den durch den Abstandhalter 3 fließenden Strom wegen des elektrischen Widerstandes des Werkstoffes des Abstandhalters 3 (dieser ist ein elektrisch leitender Werkstoff) direkt erwärmt wird. Das Erwärmen wird soweit fortgesetzt, bis die Kleberstränge 4 die für das Aktivieren des Klebers erforderliche Temperatur erreicht haben. Dann werden, gegebenenfalls nach dem Einfüllen von einem von Luft verschiedenen Gas in den Raum in der Presse, die Glasscheiben 2 an die Seitenflächen des Abstandhalters 3 bzw. die dort vorgesehenen Kleberstränge 4 aus Kleber angedrückt und nach dem Abkühlen des Klebstoffes der Isolierglasrohling aus der Presse entfernt.

Der Isolierglasrohling 6 wird dann, wie für das Herstellen von Isolierglas üblich, zu einer Versiegelungsstation gefördert, in der die nach außen offene Randfuge 5 des Isolierglasrohlings mit einer Versiegelungsmasse, beispielsweise einer Masse auf Polysulfidbasis, ausgefüllt wird.

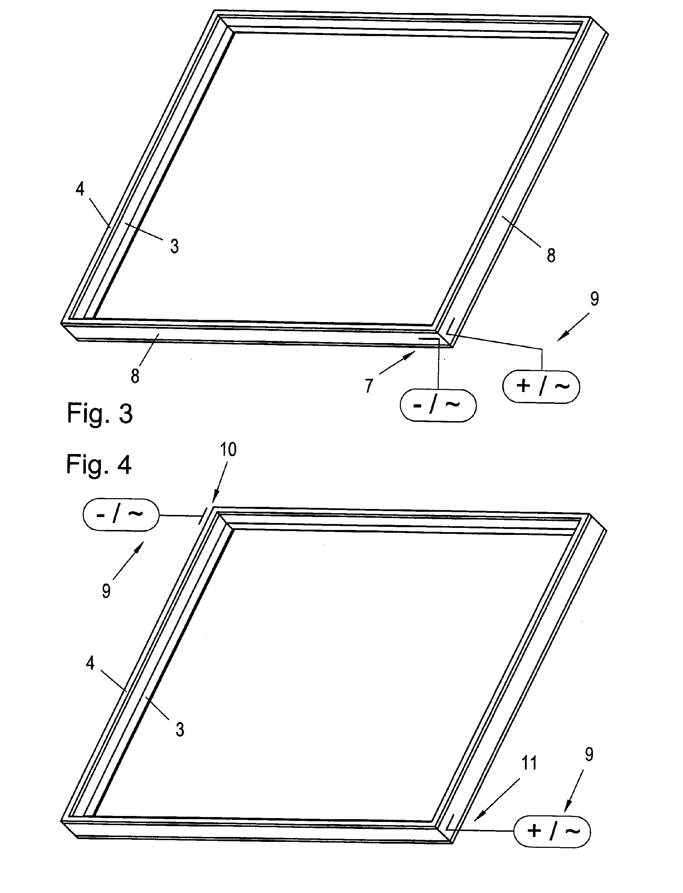

Bei der in Fig. 3 gezeigten Ausführungsform ist der Abstandhalter 3 zu einem Rahmen gebogen, der an einer Ecke 7 noch offen, oder dort durch einen Strom nicht leitenden (elektrisch isolierenden) Eckverbinder (beispielsweise ein Eckwinkel aus Kunststoff) verbunden ist. Im Bereich der Ecke 7, in der die beiden zu dieser Ecke 7 führenden Schenkel 8 des (rahmenförmigen)
Abstandhalters 3 elektrisch leitend nicht verbunden, also voneinander elektrisch isoliert sind, wird der Abstandhalter 3, wie oben erläutert, an eine Stromquelle 9 angeschlossen (Gleichstrom oder Wechselstrom), sodass der Abstandhalterrahmen zum Aktivieren der auf ihn aufgetragenen Kleberstränge 4 aus wärmeaktivierbaren Kleber (Hot-Melt) erwärmt werden kann.

Bei der in Fig. 4 gezeigten Variante ist der Abstandhalter 3 ein Rahmen, der an allen vier Ecken geschlossen ist, wobei die Ecken beispielsweise durch einstückiges Abbiegen einer Abstandhalterprofilleiste hergestellt worden sein können. In diesem Fall wird an einander gegenüberliegenden Ecken 10, 11 (diagonal gegenüberliegenden Ecken) Spannung angelegt, sodass der Abstandhalter 3 durch den durch ihn fließenden Strom nach Art einer Widerstandsheizung erwärmt wird, um den Kleber der auf ihn aufgetragenen Kleberstränge 4 zu aktivieren.

Die im Rahmen der vorliegenden Erfindung verwendbaren, wärmeaktivierbaren Klebstoffe sind unter der Bezeichnung Hot-Melt in der Isolierglasbranche bekannt. Dabei handelt es sich typischerweise um einen thermoplastischen Dichtstoff, der bei hohen Temperaturen (150 bis 180°C) verarbeitet werden kann.

In Betracht gezogen sind im vorliegenden Fall auch Butyl-basierte Schmelzkleber ebenso wie lösungsmittelfreie Hot-Melt-Kleber auf Basis von Butylkautschuk.

Zusammenfassend kann ein Ausführungsbeispiel der Erfindung wie folgt beschrieben werden:
Um beim Zusammenbauen von Isolierglasrohlingen 6, bei welchen der Abstandhalter 3 beidseitig mit einem Strang 4 aus einem wärmeaktivierbaren Kleber belegt ist, auszuführen, wird der Kleber dadurch auf seine Aktivierungstemperatur erwärmt, dass der Abstandhalter 3 durch Anlegen von Spannung nach Art einer elektrischen Widerstandsheizung erwärmt wird.

## Patentansprüche

1. Verfahren zum Zusammenbauen von Isolierglasrohlingen, bei dem Glastafeln mit einem Abstandhalter über einen wärmeaktivierbaren Kleber verbunden werden, **dadurch gekennzeichnet, dass** der wärmeaktivierbare Kleber auf die Aktivierungstemperatur erwärmt wird, indem der Abstandhalter erwärmt wird.

2. Verfahren nach Anspruch 1, **dadurch gekennzeichnet, dass** der Abstandhalter durch Anlegen einer elektrischen Spannung nach Art einer Widerstandsheizung erwärmt wird.

3. Verfahren nach Anspruch 2, **dadurch gekennzeichnet, dass** die elektrische Spannung an zwei einander gegenüberliegenden Ecken des Abstandhalters angelegt wird.

4. Verfahren nach Anspruch 2, **dadurch gekennzeichnet, dass** die Spannung an den Enden von zwei Schenkeln des Abstandhalters, die voneinander elektrisch isoliert sind, angelegt wird.

5. Verfahren nach Anspruch 4, **dadurch gekennzeichnet, dass** die Enden der Schenkel, an welchen Spannung angelegt wird, im Bereich einer Ecke des Abstandhalters angeordnet sind.

6. Verfahren nach einem der Ansprüche 1 bis 5, **dadurch gekennzeichnet, dass** der wärmeaktivierbare Kleber ein Kleber auf Basis eines Thermoplasten, von Butylkautschuk oder von lösungsmittelfreiem Hot-Melt ist.
